Europäisches Patentamt

European Patent Office

Office européen des brevets

(11) Publication number: **0 367 355**
**A1**

## EUROPEAN PATENT APPLICATION

(21) Application number: **89202764.0**

(22) Date of filing: **01.11.89**

(51) Int. Cl.5: **C12Q 1/48**

(30) Priority: **03.11.88 NL 8802703**

(43) Date of publication of application:
**09.05.90 Bulletin 90/19**

(84) Designated Contracting States:
**AT BE CH DE ES FR GB GR IT LI LU NL SE**

(71) Applicant: **RIJKSUNIVERSITEIT LIMBURG**
**Tongersestraat 53**
**NL-6213 GA Maastricht(NL)**

(72) Inventor: **Hermens,Willem Theodoor**
**Carolusweg 16**
**NI-6247 CV Gronsveld(NL)**
Inventor: **Van Peltenburg, Henricus Gerardus**
**Jekerschans 57**
**NL-6212 GH Maastricht(NL)**

(74) Representative: **de Bruijn, Leendert C. et al**
**Nederlandsch Octrooibureau**
**Scheveningseweg 82 P.O. Box 29720**
**NL-2502 LS 's-Gravenhage(NL)**

(54) **A method for the determination of ornithine-carbamyl-transferase.**

(57) Method for the determination of ornithine-carbamyl-transferase (OCT) in biological material such as plasma and serum by
    a) treatment of the biological material with citrulline and arsenate,
    b) incubation of the mixture treated under a)
    c) stopping the incubation, and
    d) carrying out an ammonia determination; and a kit which is suitable for carrying out this method.

EP 0 367 355 A1

## Method for the determination of ornithine-carbamyl-transferase

The invention relates to a method for the determination of ornithine-carbamyl-transferase (OCT) in biological material such as plasma, serum or liver homogenates.

Ornithine-carbamyl-transferase (OCT), which is also termed ornithine-transcarbamylase, is an enzyme which is formed in the liver and plays a role in the urea cycle. It catalyses the production of citrulline from ornithine, a carbamyl group being removed from carbamyl phosphate and bonded to ornithine. This enzyme is liver-specific in humans. Too large an amount of OCT in the blood plasma or serum is a clear indication of various liver disorders.

A method for the determination of the OCT content in blood serum is disclosed in a publication by R.J. Brown and S. Griseola, J. Lab. Med. (1959) 54, pp. 617-620. This method is based on the colorimetric determination of citrulline, which is formed from carbamyl phosphate and ornithine by the catalytic action of OCT. With this method the urea present in the serum, which interferes in the colorimetric determination, is decomposed with urease. However, this known method is inaccurate and cannot be carried out in a single vessel. Moreover, a urease step has to be carried out separately.

In other known methods use is made of a radioactively labelled substrate (Reichard et al., J. Lab. Clin. Med. (1958), 52, pp. 709-717), or the laborious and inaccurate microdiffusion technique has to be used, or a protein removal is necessary, which features make these methods unattractive.

According to the invention, a method as specified in the preamble has been found with which the OCT content can be determined in an accurate and simple manner, i.e. in a single vessel.

The method according to the invention is characterized in that the following steps are carried out in succession:

a) combination of the serum or plasma, in which it is desired to determine the OCT content, with citrulline and arsenate,

b) incubation of the mixture formed under a),

c) stopping the incubation under step b), and

d) carrying out an ammonia determination.

With the method according to the invention it is preferred, in step a), to use a solution of citrulline and sodium arsenate which has a pH of 7.0 - 7.2 and preferably 7.1, in step b), to carry out the incubation for 2-36 hours, and preferably 4-24 hours, at a temperature of 25-37°C and preferably 37°C, in step c), to stop the incubation by freezing and, in step d), to carry out the ammonia determination in accordance with the method of Da Fonseca-Wollheim, Z. Klin. Chem. Klin. Biochem. (1973, part 10, pp. 426-431). This determination is relatively simple and reliable.

In step a), citrulline is reacted with the arsenate to form ornithine and carbamyl arsenate, OCT acting as a catalyst. The carbamyl arsenate formed is unstable and decomposes with the formation of carbon dioxide, ammonia and arsenate. In general, a relatively long incubation, for example of 24 hours, is necessary because of the small amount of OCT in the sample.

There is a direct linear relationship between the incubation period and the ammonia production. Essentially, the incubation period is not significant provided that the calculation is adjusted to the period used. However, it is important to use a long (for example 24 hours) incubation in the case of (presumably) low OCT values (for example < 200 mU/l) since otherwise the ammonia formation is too low. For OCT activities which are (presumably) high (for example > 1500 mU/l) it will in exceptional cases be possible for a 4-hour incubation procedure to suffice (this makes the method suitable as a so-called "rapid" determination).

An alternative to stopping the incubation reaction by means of freezing is removal of proteins with the aid of trichloric acid or perchloric acid (TCA/PCA). However, the number of extra steps (protein removal; centrifuging; neutralizing; placing on ice for precipitation, etc.) makes this procedure laborious. The reliability of the $NH_3$ determination without protein removal is good; compare Da Fonseca-Wollheim, loc. cit.

In step d) the amount of ammonia formed is measured, preferably with the aid of an enzymatic method, for example in accordance with the method of Da Fonseca-Wollheim, loc. cit. With this method the decrease in the extinction at 340 nm is a measure for the ammonia concentration in the sample.

Moreover, any other method for the determination of ammonia in step d) can be chosen; compare M.A. Janssen, C.L.H. of Berlo, P.A.M. of Leeuwen, and P.B. Soeters "The determination of ammonia in plasma and whole blood" in: P.B. Soeters et al., Ed. Advances in ammonia metabolism and hepatic encephalopathy, Amsterdam: Elsevier Sc.Publ., 1988 Ch 79, 1-6.

The analytical method according to the invention can be carried out both with human and with non-human material. With this method the ammonia determination (step d) is not influenced by the incubation step (step b).

The invention also relates to a kit which is suitable for carrying out the method according to the invention. The kit according to the invention is characterized in that it contains the following constituents:
- arsenate buffer,
- L-citrulline solution,
- means for carrying out an ammonia determination.

For the benefit of the user, the kit for carrying out the determination includes a description.

The following review relates to a kit for approximately 200 determinations.

Reagents:

For the incubation step:

- 0.5 M sodium arsenate buffer, pH 7.1 (20 ml): 3.12g of sodium arsenate are dissolved in 16 ml of water, the pH is adjusted to 7.1 with 2 N HCl and the volume is made up to 20 ml,
- 0.1 M L-citrulline arsenate buffer: 175 mg of L-citrulline are dissolved in 10 ml of the abovementioned sodium arsenate buffer.

Preparation of reagents:

1. 0.15 mol/l triethanolamine buffer of pH 8.6

Dissolve 27.86 g of triethanolamine hydrochloride (M = 185.7) in ammonia-free water. Adjust the pH to 8.6 and make up to 1 litre.

2. 0.4 mol/l α-ketoglutaric acid

Dissolve 292 mg of α-ketoglutaric acid in 3.6 ml of 1N NaOH (M = 146.1), adjust the pH to 7.4 and make up to 5 ml with ammonia-free water.

3. 20 mol/l ADP

Dissolve 94.2 mg of ADP (molecular weight 471.2) in 10 ml of ammonia-free water.

4. 4 mmol/l NaDPH

Dissolve 20 mg of NaDPH in 5 ml of 0.12 mol/l NaHCO$_3$.

5. 10 mg/kl glutamate dehydrogenase (GLDH)

Solution in 50 % glycerol, ready-for-use.

Standard solutions for the centrifugal analyser.

To prepare a stock solution 330 mg of dried ammonium sulphate are dissolved in 1 litre of twice distilled water. This gives a 5 mM ammonia solution. This solution is then diluted to 300, 400, 500, 1000 and 2000 μM. These solutions of different concentrations are needed because the centrifugal analysis apparatus used operates with calibration standards. Initially NH$_3$ is measured in a standard solution and NH$_3$ is then measured in the sample to be investigated. The extinction found for the sample is then extrapolated in the calibration curve found. The use of relatively high and low ammonia concentrations is the consequence of the wide range in OCT activities (100-10,000 mU/l). The final curve was obtained using a OCT preparation from Streptococcus fecalis, a lyophilized powder which contains 40 % protein and 835 units OCT activity per mg of protein.

Description of the method.

I - Manual method

A: incubation step

50 $\mu$l of plasma are placed in each of two cuvettes (1 = test, 2 = blank). 50 $\mu$l of citrulline arsenate buffer are added to cuvette 1 and 50 $\mu$l of arsenate buffer are added to cuvette 2. The cuvettes are closed and the contents mixed with the aid of a vortex mixer. The mixtures are incubated for 24 hours at 37° C. The incubated material can be subjected to an ammonia determination immediately or can be stored at -70° C.

B: enzymatic ammonia determination

Frozen samples are thawed for 3 minutes at 37° C and mixed with the aid of a vortex mixer. 750 $\mu$l of a reaction mixture of the following (abovementioned) components:
20 ml of triethanolamine buffer
1 ml of $\alpha$-ketoglutaric acid solution
2 ml of ADP solution
1 ml of NaDPH solution
6 ml of ammonia-free water
are added to the incubation cuvette.
Final concentration of the ready-to-use reagents: (after the components have been added in the volumes indicated):
triethanolamide HCl 100 mmol/l, pH 8.6
$\alpha$-ketoglutarate 13.33 mmol/l
ADP 1.33 mmol/l
NaDPH 0.133 mmol/l.
150 $\mu$l of twice distilled water are also added. The whole is mixed and the extinction E1 is read off after 10 minutes.
10 $\mu$l of the following GLDH solution are added to the reaction mixture: GLDH from bovine liver (Boehringer article no. 127 710 (100 mg/10 ml)), solution in 50 % glycerol, pH approximately 7, $NH_4$-free.
The whole is mixed again and the extinction E2 is read off after 10 minutes.
A further 10 $\mu$l of the same GLDH solution are then added.
The whole is mixed again and the extinction E3 is read off after 10 minutes.

Calculation

The $NH_3$ concentration in the sample is:

$$\frac{1000 \times total\ volume \times (E_1 - E_2) - (E_2 - E_3)}{6.22 \times 1.0 \times volume\ of\ the\ sample} = 3280 \times \Delta E\ \mu mol/l$$

The extinction is read off at 340 nm.
At concentrations above 400 $\mu$mol/l the mixture is diluted with ammonia-free water.

4

II - Determination with the aid of the centrifugal analysis equipment (centrifugal analyser)

A: incubation step:

As above under I

B: enzymatic ammonia determination

Frozen samples are thawed for 3 minutes at 37°C and mixed with the aid of a vortex mixer. Concentrations of 300, 400 and 500 $\mu$mol/l are used for the calibration standards. The GLDH solution is diluted 1:10 (starting reagent). The ammonia determination is carried out in accordance with the method of Da Fonseca Wollheim (ref.). For this method the analysis equipment is adjusted in accordance with the following parameter listing:

| PARAMETER LISTING | |
| --- | --- |
| a KEY 13-0-41-53-53-55-54-49-51 | |
| | $\mu$mol/l (4) |
| 1) Units | |
| 2) Calculation factor | 0 |
| 3) Standard 1 conc | 300 |
| 4) Standard 2 conc | 400 |
| 5) Standard 3 conc | 500 |
| 6) Limit | 500 |
| 7) Temperature (°C) | 30.0 |
| 8) Type of analysis | 6 |
| 9) Wavelength (nm) | 340 |
| 10) Sample volume ($\mu$l) | 50 |
| 11) Diluent volume ($\mu$l) | 30 |
| 12) Reagent volume ($\mu$l) | 300 |
| 13) Incubation time (sec) | 300 |
| | $\mu$mol/l (4) |
| 14) Start reagent volume ($\mu$l) | 20 |
| 15) Time of first reading (sec) | 300.0 |
| 16) Time interval (sec) | 300 |
| 17) Number of readings | 01 |
| 18) Blanking mode | 1 |
| 19) Printout mode | 1 |

The OCT activity is expressed in mU/l.

1 U/l = 1 $\mu$mol/l substrate conversion or product formation per minute (in general at 37°C)

1 mU/l = 1 nmol/l substrate conversion or product formation per minute at 37°C

1 $\mu$mol/l NH$_3$/24 hours corresponds to 1000/1440 = 0.694 nmol/l NH$_3$/minute = 0.694 mU/l OCT or vice versa.

1 mU/l OCT corresponds to 1.44 $\mu$mol/l NH$_3$ formation per 24 hours.

Calculation of OCT from the NH$_3$ concentration found.

The OCT activity of both the citrulline-containing test-tube and the blank tube is determined. The result of the NH$_3$ determination is multiplied by 2 and by 0.694 and gives the OCT activity in mU/l. The net OCT activity in mU/l is obtained by deducting the value for the blank tube from the value for the tube containing citrulline.

This calculation is explained in more detail with the aid of the following example:

ammonia in test sample = 857 μmol/l/24 hours
ammonia in blank sample (contains plasma and all reagents except for citrulline) = 121 μmol/l/24 hours
ammonia as a result of OCT activity 736 μmol/l/24 hours
x 2 = 1472 μmol/l/24 hours (correction 1:1 dilution)
x 0.694 = 1022 nmol/l/min
= 1022 mU/l

The normal value for the OCT activity in plasma is approximately 4-95 mU/l. In the case of a disorder values of 100-10.000 mU/l are found.

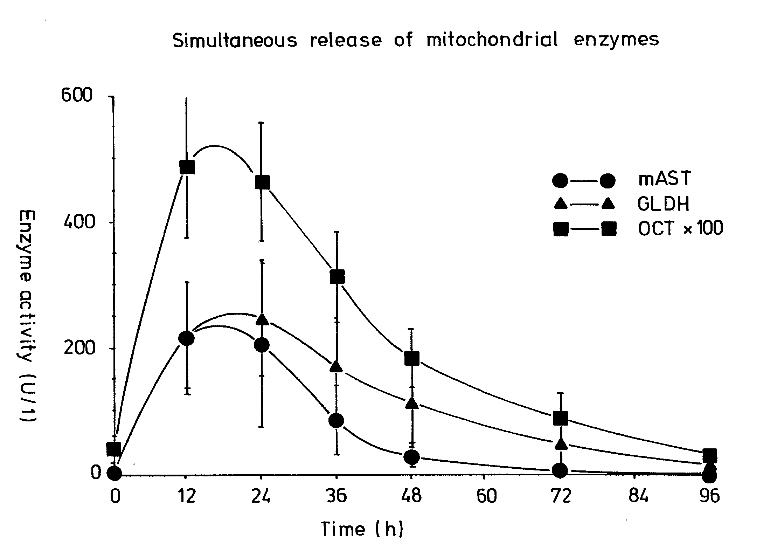

In the appended figure the plasma enzyme activity of three mitochondrial liver enzymes, OCT, mAST and GLDH, measured in four patients with acute liver damage, are compared. It is clearly seen that the release of these mitochondrial enzymes takes place at the same time and that OCT is removed slowly from the plasma, in particular in comparison with mAST.

In this figure the various activities were determined as follows:
- OCT using the method described above;
- total AST by means of a commercially available kit (Boehringer No. B258784); iso-enzyme separation took place by means of gel electrophoresis (on LD gels using a Beckman electrophoresis apparatus);
- GLDH using a commercially available kit (Boehringer No. 124320).

OCT can advantageously be used in the determination of hepatic necrosis. The combination of the incubation method with an enzymatic ammonia analysis, which is employed with the method according to the invention, makes the OCT determination easy and suitable for analyses to be carried out on a routine basis.

## Claims

1. Method for the determination of ornithine-carbamyl-transferase (OCT) in biological material such as plasma, serum and liver homogenates, with which the following steps are carried out successively:
a) combination of the serum or plasma, in which it is desired to determine the OCT content, with citrulline and arsenate,
b) incubation of the mixture formed under a),
c) stopping the incubation under step b), and
d) carrying out an ammonia determination.
2. Method according to Claim 1, in which
in step a) a solution of citrulline and sodium arsenate with a pH of 7.O-7.2 is used,
in step b) the incubation is carried out for 12-36 hours and preferably 24 hours at a temperature of 25-37° C and preferably 37° C,
in step c) the incubation is stopped by freezing, and in step d) the ammonia determination is carried out in accordance with the method of Da Fonseca-Wollheim, Z. Klin. Chem. Klin. Biochem. (1973, part 10, pp. 426-431).
3. Kit suitable for carrying out the method according to Claim 1 or 2, which contains the following constituents
- arsenate buffer
- L-citrulline solution
- means for carrying out an ammonia determination.
4. Kit according to Claim 3 as described in the preceding description.

Simultaneous release of mitochondrial enzymes

mAST
GLDH
OCT × 100

Enzyme activity (U/1)

Time (h)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D | JOURNAL OG LAB. & CLIN. MED., vol. 52, November 1958, pages 709-717; H. REICHARD et al.: "Determination of ornithine carbamyl transferase in serum" * Whole document * | 1,3 | C 12 Q 1/48 |
| Y | IDEM --- | 2,4 | |
| X | CLINICAL CHEMISTRY, vol. 28, no. 7, 1982, page 1601, no. 260; J.I. JARZABEK et al.: "Ornithine carbamyl transferase (OCT) determination using the Du Pont ACA ammonia method" * Whole document * --- | 1,3 | |
| Y,D | Z. KLIN. CHEM. KLIN. BIOCHEM., vol. 11, no. 10, 1973, pages 426-431; F. DA FONSECA-WOLLHEIM: "Direkte Plasmaammoniakbestimmung ohne Enteiweissung" * Pages 426-427 * ------ | 2,4 | |

TECHNICAL FIELDS SEARCHED (Int. Cl.5)

C 12 Q

The present search report has been drawn up for all claims

| Place of search | Date of completion of the search | Examiner |
|---|---|---|
| THE HAGUE | 03-01-1990 | HITCHEN C.E. |